(19) Europäisches Patentamt
European Patent Office
Office européen des brevets

(11) Numéro de publication: **0 086 678**
**B1**

(12) # FASCICULE DE BREVET EUROPEEN

(45) Date de publication du fascicule du brevet: **03.07.85**

(51) Int. Cl.⁴: **C 07 D 471/04**, C 07 D 241/08 // (C07D471/04, 241:00, 221:00)

(21) Numéro de dépôt: **83400044.0**

(22) Date de dépôt: **07.01.83**

(54) **Procédé pour la préparation d'acyl-2 hexahydro-1,3,4,6,7,11b-2H-pyrazino (2,1-a)isoquinoléinones-4 et intermédiaires.**

(30) Priorité: **29.01.82 FR 8201758**

(43) Date de publication de la demande: **24.08.83 Bulletin 83/34**

(45) Mention de la délivrance du brevet: **03.07.85 Bulletin 85/27**

(84) Etats contractants désignés: **AT BE CH DE FR GB IT LI LU NL SE**

(56) Documents cités: **CH - A - 479 594**
**DE - A - 2 457 971**

(73) Titulaire: **SANOFI, Société dite:, 40, Avenue George V, F-75008 Paris (FR)**

(72) Inventeur: **Frehel, Daniel Résidence le Grand Ramier, Bat. C, Appt. 52-7 Avenue Pr. C. Soula, F-31400 Toulouse (FR)**
Inventeur: **Maffrand, Jean-Pierre, 5 Rue du Corps-Franc-Pommiès, F-31120 Portet/Garonne (FR)**

(74) Mandataire: **Polus, Camille et al, c/o Cabinet Lavoix 2, Place d'Estienne d'Orves, F-75441 Paris Cedex 09 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention concerne un procédé pour la préparation d'acyl-2 hexahydro-1,3,4,6,7,11b 2H-pyrazino [2,1-a]-isoquinoléinones-4.

Plus particulièrement, la présente invention a pour objet un nouveau procédé pour la préparation de composés de formule:

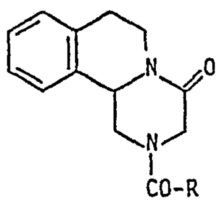

(I)

dans laquelle R représente un groupe alkyle inférieur, cycloalkyle ayant 3 à 8 atomes de carbone, phényle ou phényle substitué.

Les acyl-2 hexahydro-1,3,4,6,7,11b 2H-pyrazino [2,1-a]-isoquinoléinones-4 de formule I ci-dessus sont des produits bien connus qui sont décrits dans les brevets allemands Nos 1795728 et 2362539 comme puissants anthelminthiques ou comme intermédiaires dans la préparation de produits à activité anthelminthique.

Le composé de formule I ci-dessus où R est un groupe cyclohexyle a reçu la dénomination commune internationale «praziquantel» et il a été indiqué comme ayant un large spectre anthelminthique avec une activité excellente contre toutes les espèces de Schistosomes pathogènes chez l'homme et les Cestodes (Experientia 1977, 1036).

Les brevets allemands Nos 2362539 et 2441261 décrivent un procédé qui peut être utilisé pour la préparation des composés de formule générale I ci-dessus, ledit procédé étant caractérisé en ce que l'on traite l'oxo-4 hexahydro-1,2,3,6,7,11b 2H-pyrazino [2,1-a]-isoquinoléine de formule:

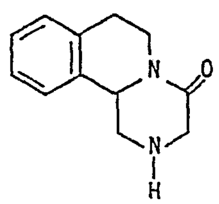

(II)

avec l'acide R-COOH, ou avec un de ses dérivés fonctionnels, ou bien que l'on traite un composé de formule:

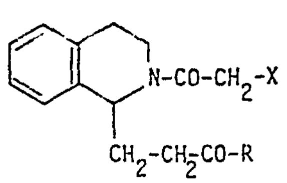

(III)

dans laquelle X représente un halogène, un groupe méthylsulfonyloxy ou un groupe arylsulfonyloxy contenant 6 à 10 atomes de carbone, en présence d'un agent de cyclisation, ou encore, quand il s'agit de préparer des diastéréo-isomères, que l'on traite avec un agent de réduction un composé de formule:

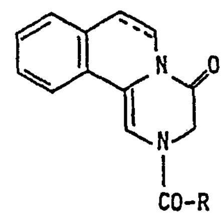

(IV)

dans laquelle la ligne en pointillé indique qu'il peut y avoir une double liaison en position 6,7.

Selon le brevet allemand No 2504250, le produit de départ III présente l'inconvénient de n'être accessible qu'avec des rendements très faibles à partir de l'isoquinoléine, du chlorure d'acide R-COCl et des cyanures.

Le même brevet vise un procédé caractérisé en ce que l'on traite un composé de formule:

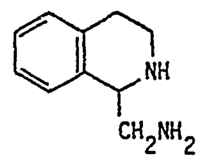

(V)

sous forme de mono-sels d'addition, avec le chlorure R-COCl en présence d'une base faible qui est plus faiblement basique que le produit que l'on obtient, et on transforme ensuite le composé obtenu en le composé III ci-dessus par action d'un halogénure d'halogénoacétyle.

Les composés III sont ensuite transformés en les composés de formule I ci-dessus selon le procédé du brevet allemand No 2362539.

Le composé de formule V est à son tour préparé par hydrogénation catalytique du composé de formule:

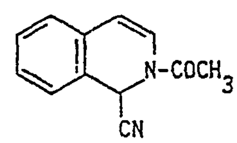

(VI)

en utilisant de préférence le Ni Raney comme catalyseur (Helv. Chim. Acta, 1939, *22*, 673-683), puis par hydrolyse. Cette hydrogénation comporte cependant, déjà pour des quantités de 150 à 200 mg, une opération à haute température et à une pression très élevée (d'au moins 150 jusqu'à 250 atmosphères), ce qui constitue un inconvénient sur le plan industriel, surtout quand les quantités sont encore plus importantes.

Le brevet allemand No 2457971 décrit un autre procédé pour la préparation des composés de formule I ci-dessus qui consiste à cycliser un composé de formule:

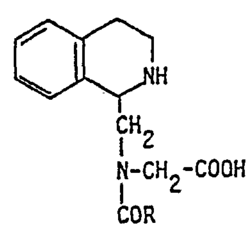

(VII)

ou un de ses dérivés fonctionnels sur le carboxyle.

Les composés VII sont cependant préparés à partir du produit V ci-dessus qui demande, pour son obtention, des températures et des pressions élevées.

Selon le même brevet, on peut cycliser aussi un composé de formule:

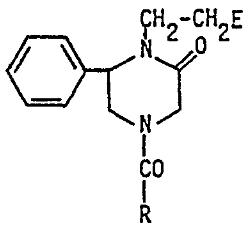

(VIII)

où E est un halogène ou un groupe hydroxyle, mais cette procédure n'est pas la préférée par rapport à celle qui comporte la cyclisation du produit VII.

De toutes façons, toutes les méthodes qui conduisent aux produits de formule I avec des rendements convenables, comportent, en amont, soit une hydrogénation à des pressions et des températures très élevées, en présence de catalyseurs tels que le Ni Raney, qui nécessitent une préparation délicate, soit d'autres procédés qui comportent, par exemple, l'utilisation de gaz fluorhydrique ou d'intermédiaires siliciés coûteux.

Il a été maintenant trouvé que les composés de formule générale I ci-dessus peuvent être préparés avec des rendements globaux élevés par le procédé de la présente invention qui fait appel à de nouveaux intermédiaires et à des réactions chimiques faciles à mettre en œuvre, dont la transposition à l'échelle industrielle est aisée.

Ainsi, selon un de ses aspects, la présente invention concerne un procédé pour la préparation d'acyl-2 hexahydro-1,3,4,6,7,11b 2H-pyrazino [2,1-a]-isoquinoléinones-4 de formule I ci-dessus, ledit procédé étant caractérisé en ce que l'on traite une acyl-4 dioxo-2,6 pipérazine de formule:

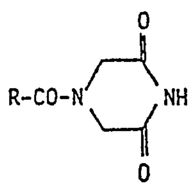

(IX)

avec un agent alkylant de formule:

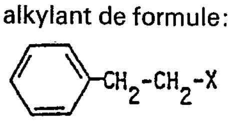

(X)

où X représente le chlore, le brome, l'iode ou un groupe nucléofuge, en présence d'un agent de condensation alcalin dans un solvant organique inerte; on réduit sélectivement un des carbonyles imidiques du composé ainsi obtenu de formule:

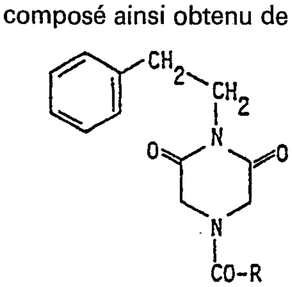

(XI)

par un hydrure métallique complexe en présence d'un chlorure métallique catalyseur choisi parmi $CuCl_2$, $CoCl_2$, $NiCl_2$, $CrCl_3$, $FeCl_3$, $SnCl_2$, $SrCl_2$, $MnCl_2$, $CeCl_2$, $HgCl_2$, et on cyclise le composé ainsi obtenu de formule:

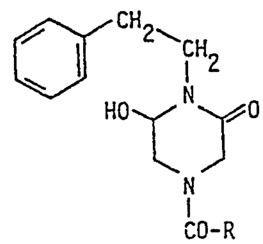

(XII)

par action d'un acide minéral ou organique fort à une température comprise entre 0 et 25° C.

Selon un autre de ses aspects, la présente invention a pour objet, en tant qu'intermédiaires, de nouvelles acyl-4 oxo-6 phénéthyl-1 pipérazines de formule:

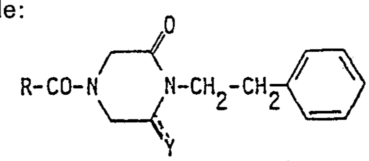

(XIII)

où R est tel que défini ci-dessus et où Y est O ou H,OH.

La configuration stérique de l'hydroxyle dans la position 2 des composés XII ci-dessus n'a pas d'importance dans le procédé de la présente invention car la cyclisation passe par un carbocation qui détruit le centre asymétrique en 2 et les produits finaux sont obtenus avec un rendement pratiquement quantitatif.

Le terme «alkyle inférieur», tel qu'utilisé ici, désigne un radical hydrocarboné aliphatique saturé à chaîne droite ou ramifiée contenant jusqu'à 6 atomes de carbone tel que méthyle, éthyle, propyle, isopropyle, isobutyle, sec-butyle, tert-butyle et similaires.

Le terme «phényle substitué», tel qu'utilisé ici, désigne un groupe phényle mono- ou disubstitué par un atome ou un radical inerte dans les conditions de réaction ci-dessus, tels qu'un halogène comme le fluor, le chlore ou le brome, un groupe hydroxy libre ou éthérifié avec un alkyle inférieur tel que défini ci-dessus, un groupe amino libre ou mono- ou disubstitué par un alkyle inférieur tel que défini ci-dessus, un groupe mercapto libre ou thioéthérifié avec un groupe alkyle inférieur tel que défini ci-dessus, un groupe cyano, un groupe trifluorométhoxy ou trifluorométhylthio.

La réaction entre le composé de départ de formule IX ci-dessus et le réactif de formule X est une alkylation conduite en présence d'un agent de condensation alcalin tel qu'un hydrure de métal alcalin, par exemple de sodium, un alcoolate alcalin et similaires. Le groupe nucléofuge indiqué par X dans la formule X ci-dessus, est de préférence le groupe méthanesulfonyloxy (mésyloxy) ou p-toluènesulfonyloxy (tosyloxy), mais les groupes benzènesulfonyloxy, β-naphtalènesulfonyloxy et similaires peuvent être également utilisés. Les agents alkylants particulièrement préférés sont le bromure de phénéthyle et l'iodure de phénéthyle.

Selon un mode opératoire préférentiel, on chauffe le mélange réactionnel pendant 2 à 8 h à une température comprise entre 50 et 100° C dans un solvant organique inerte tel que le diméthylformamide, le diméthylacétamide, le dioxanne ou le diméthoxyéthane. A la fin de la réaction, le produit ainsi obtenu est isolé selon la technique conventionnelle et il peut être purifié ou utilisé directement pour l'étape suivante.

L'acyl-4 dioxo-2,6 phénéthyl-1 pipérazine de formule XI est ensuite réduite avec un hydrure métallique complexe, comme l'hydrure double d'un métal alcalin et d'un métal alcalino-terreux tel que le borohydrure de sodium, le borohydrure de lithium ou l'hydrure de lithium et d'aluminium ou bien d'autres hydrures métalliques complexes tels que le borohydrure de potassium et de tri-sec.-butyle en présence de chlorure métallique catalyseur. On opère en général dans un solvant inerte comme un éther, tel que l'éther diéthylique, le tétrahydrofuranne, le dioxanne ou le diméthoxyéthane à une température comprise entre −70° C et la température d'ébullition du solvant employé. Si on utilise le borohydrure de sodium, la réaction peut être conduite en solution aqueuse ou hydroalcoolique à une température proche de la température ambiante.

Selon un mode opératoire préférentiel, on opère avec un excès de borohydrure de sodium dans un solvant alcoolique tel que le méthanol ou l'éthanol à une température comprise entre 0 et 25° C en utilisant comme catalyseur le chlorure cuivrique ($CuCl_2$). Après avoir détruit l'excès d'agent réducteur, le composé ainsi obtenu, l'acyl-4 hydroxy-2 oxo-6 phénéthyl-1 pipérazine de formule XII, est isolé selon les méthodes conventionnelles et purifié ou bien utilisé tel quel pour la cyclisation.

La cyclisation est effectuée par traitement avec un acide minéral ou organique fort tel que l'acide chlorhydrique, l'acide sulfurique, l'acide phosphorique, l'acide trifluoroacétique et similaires à une température comprise entre 0 et 25° C, de 0 à 5° C de préférence. Le produit final de formule I ci-dessus est isolé en versant le mélange réactionnel dans de l'eau glacée et par extraction avec un solvant approprié.

Les acyl-4 dioxo-2,6 pipérazines de formule IX utilisées comme composé de départ dans le procédé de la présente invention sont des produits connus dans la littérature ou facilement préparés à partir de l'iminodiacétonitrile par réaction avec un halogénure d'acide, par traitement ultérieur du N-acyl-iminodiacétonitrile ainsi obtenu avec l'hydroxylamine et diazotation selon le schéma suivant:

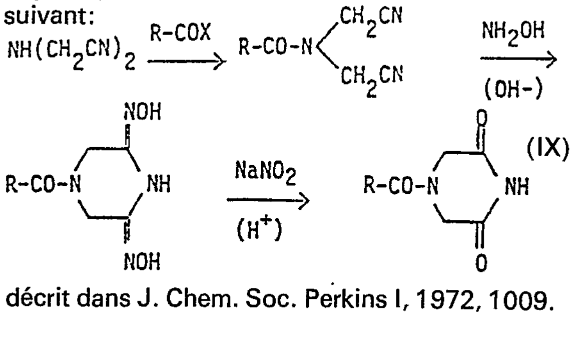

(IX)

décrit dans J. Chem. Soc. Perkins I, 1972, 1009.

Les acyl-4 dioxo-2,6 pipérazines de formule IX utilisées comme composés de départ peuvent également être préparées en faisant réagir un acide N-acyl-iminodiacétique ou la diamine correspondante avec le formamide tout en éliminant l'eau qui se forme par voie azéotropique selon le schéma suivant:

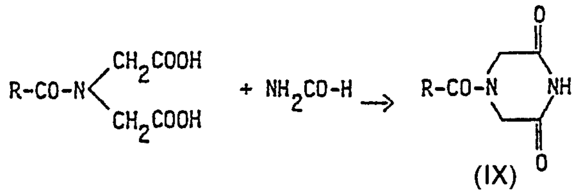
(IX)

comme décrit dans le brevet belge N° 624686.

Le exemples suivants illustrent l'invention.

*Préparation:*

a) *N-(cyclohexylcarbonyl)-iminodiacétonitrile*

On mélange sous agitation vigoureuse 50 g (0,52 mol) d'iminodiacétonitrile, 160 g (0,78 mol) de carbonate de potassium, 150 ml d'eau et 400 ml de dichlorométhane. Après refroidissement du milieu réactionnel à 0° C, on ajoute, goutte à goutte, 96,5 g (0,65 mol) de chlorure de cyclohexylcarbonyle. On laisse revenir à la température ambiante en maintenant l'agitation pendant 2 h. On décante la phase organique, et on extrait la phase aqueuse deux fois avec du dichlorométhane. Les phases organiques réunies, séchées sur sulfate de sodium, laissent un résidu huileux après évaporation. Par trituration avec du cyclohexane, on récupère des cristaux que l'on recristallise dans le cyclohexane. Cristaux blancs, F = 82° C, rendement: 85%.

b) *(Cyclohexylcarbonyl)-4 bis-(hydroxyimino)-2,6 pipérazine*

A un mélange de 19,4 g (0,28 mol) de chlorhydrate d'hydroxylamine et de 7,42 g (0,07 mol) de carbonate de potassium dans 60 ml de méthanol et 20 ml d'eau, on ajoute par portions 14,4 g (0,07 mol) de N-(cyclohexylcarbonyl)-iminodiacétonitrile. On porte à reflux, sous atmosphère inerte, pendant 2 h 30. Après refroidissement, on filtre les cristaux. On met en suspension ces cristaux dans un mélange de 120 ml d'éthanol et 50 ml d'eau et on porte à reflux pendant 1 h. On filtre à chaud les cristaux que l'on recristallise dans un mélange d'eau et d'éthanol. Cristaux blancs, F > 260° C, rendement: 84%.

c) *(Cyclohexylcarbonyl)-4 dioxo-2,6 pipérazine*

On met en suspension 6 g (0,023 mol) de (cyclohexylcarbonyl)-4 bis-(hydroxyimino)-2,6 pipérazine dans un mélange de 20 ml d'eau et 20 ml d'acide acétique. Après refroidissement du milieu réactionnel entre 0 et 5° C, on ajoute, goutte à goutte, sous atmosphère inerte, une solution de 5 g (0,069 mol) de nitrite de sodium dans 25 ml d'eau. On abandonne 24 h à température ambiante. L'évaporation à sec laisse un résidu que l'on purifie par filtration sur un lit de silice (éluant: dichlorométhane/méthanol 9/1). Les cristaux obtenus sont recristallisés dans l'éthanol. Cristaux blancs, F = 180° C, rendement: 83%.

De la même façon, en faisant réagir l'iminodiacétonitrile avec le chlorure de cyclopropylcarbonyle, le chlorure de cyclobutylcarbonyle, le chlorure de cyclopentylcarbonyle, le chlorure de cycloheptylcarbonyle et, respectivement, le chlorure de cyclooctylcarbonyle, en traitant les produits ainsi obtenus par du chlorhydrate d'hydroxylamine et en soumettant les dioximes à une diazotation selon le mode opératoire décrit ci-dessus, on obtient la (cyclopropylcarbonyl)-4 dioxo-2,6 pipérazine, la (cyclobutylcarbonyl)-4 dioxo-2,6 pipérazine, la (cyclopentylcarbonyl)-4 dioxo-2,6 pipérazine, la (cycloheptylcarbonyl)-4 dioxo-2,6 pipérazine et, respectivement, la (cyclooctylcarbonyl)-4 dioxo-2,6 pipérazine.

*Exemple 1:*

a) *(Cyclohexylcarbonyl)-4 dioxo-2,6 phénéthyl-1 pipérazine*

On dissout 15 g (0,067 mol) de (cyclohexylcarbonyl)-4 dioxo-2,6 pipérazine dans 200 ml de diméthylformamide. On ajoute 4 g (0,073 mol) de méthylate de sodium et on abandonne pendant 1 h à température ambiante. Au milieu réactionnel on ajoute, goutte à goutte, sous atmosphère inerte, une solution de 19 g (0,081 mol) d'(iodo-2 éthyl) benzène dans 50 ml de diméthylformamide et on porte à 80° C pendant 5 h. On évapore à sec, sous vide, et reprend le résidu par un mélange d'eau et de dichlorométhane. La phase organique décantée est séchée sur sulfate de sodium sec. L'évaporation du solvant laisse un résidu huileux que l'on purifie par chromatographie sur colonne de silice (éluant: toluène/acétate d'éthyle 7/3). Les cristaux obtenus se recristallisent dans le cyclohexane. Cristaux blancs, F = 90° C, rendement: 86%.

b) *(Cyclohexylcarbonyl)-4 hydroxy-2 oxo-6 phénéthyl-1 pipérazine*

On dissout 15,2 g (0,046 mol) de (cyclohexylcarbonyl)-4 dioxo-2,6 phénéthyl-1 pipérazine dans 600 ml d'éthanol. Sous atmosphère inerte, à 0° C, on ajoute 8,7 g (0,0509 mol) de chlorure cuivrique dihydraté et on abandonne pendant 1 h à 0° C. Par portions, on ajoute au milieu réactionnel, maintenu à 0° C, 8,8 g (0,23 mol) de borohydrure de sodium et on abandonne 45 min à 0° C. L'excès de réducteur est détruit par addition d'acétone. On filtre les sels insolubles et on évapore à sec le filtrat. Le résidu obtenu est traité par un mélange d'eau et de dichlorométhane. On décante la phase organique, on la sèche sur sulfate de sodium sec et on évapore à sec. Le résidu obtenu se purifie par filtration sur un lit de silice (éluant: acétate d'éthyle). Cristaux beiges, F = 134° C, rendement: 73%.

c) *(Cyclohexylcarbonyl)-2 hexahydro-1,3,4,5,6,7,11b 2H-pyrazino (2,1-a) isoquinoléinone-4 (praziquantel)*

On ajoute par portions 12,25 g (0,037 mol) de (cyclohexylcarbonyl)-4 hydroxy-2 oxo-6 phénéthyl-1 pipérazine à 150 ml d'acide chlorhydrique 12N préalablement refroidi à 0° C et on abandonne pendant une nuit à la température ambiante. On verse le milieu réactionnel dans l'eau glacée et on extrait avec du dichlorométhane. La phase organique séchée sur du sulfate de sodium sec, et évaporée à sec, laisse une huile qui cristallise lentement au repos. Les cristaux de praziquantel ainsi obtenus se recristallisent dans un mélange d'éther de pétrole et d'acétone. Cristaux blancs, F = 138-140° C, rendement: 95%. Le rendement global en praziquantel à partir de l'iminodiacétonitrile, composé de départ de la préparation, est de 35%.

*Exemple 2:*

On dissout 0,067 mol de (cyclopropylcarbonyl)-4 dioxo-2,6 pipérazine dans 200 ml de dimétylacétamide. On ajoute 0,073 mol de méthylate de sodium et on laisse le mélange 1 h à la température ambiante. Au milieu réactionnel on ajoute, goutte à goutte, sous atmosphère inerte, une solution de 0,081 mol de (tosyloxy-2 éthyl)-benzène dans 50 ml de diméthylacétamide et on porte à 90° C pendant 4 h. On évapore à sec sous pression réduite, on reprend le résidu par un mélange d'eau et de chlorure de méthylène et on sèche la phase organique décantée sur du sulfate de sodium sec. On évapore le solvant et on dissout le résidu constitué par de la (cyclopropylcarbonyl)-4 dioxo-2,6 phénéthyl-1 pipérazine brute, dans 600 ml d'éthanol. On ajoute à la solution ainsi obtenue, à 0° C et sous atmosphère inerte, 0,059 mol de chlorure de manganèse et on abandonne 1 h à 0° C. Par portions, on ajoute au milieu réactionnel, maintenu à 0° C, 0,23 mol de borohydrure de sodium et on laisse le mélange réactionnel 45 min à 0° C. L'excès de réducteur est détruit par addition d'acétone. On filtre les sels insolubles, on évapore à sec le filtrat et on traite le résidu par du mélange d'eau et de chlorure de méthylène. On sèche la phase organique décantée sur du sulfate de sodium sec, on évapore à sec. On purifie le résidu par filtration sur un lit de silice et on ajoute par portions le produit ainsi obtenu, constitué par la (cyclopropylcarbonyl)-4 hydroxy-2 oxo-6 phénéthyl-1 pipérazine, à 150 ml d'acide chlorhydrique préalablement refroidi à 0° C. Après une nuit à température ambiante, le milieu réactionnel est versé dans de l'eau glacée et extrait avec du dichlorométhane. On sèche la phase organique sur du sulfate de sodium anhydre, on l'évapore. On obtient ainsi le (cyclopropylcarbonyl)-2 hexahydro-1,3,4,6,7,11b 2H-pyrazino (2,1-a) isoquinoléinone-4; F = 148-149° C, rendement: 57%.

De la même façon, de la (cyclobutylcarbonyl)-4 dioxo-2,6 pipérazine, de la (cyclopentylcarbonyl)-4 dioxo-2,6 pipérazine, de la (cycloheptylcarbonyl)-4 dioxo-2,6 pipérazine et, respectivement, de la (cyclooctylcarbonyl)-4 dioxo-2,6 pipérazine, on obtient:

la (cyclobutylcarbonyl)-2 hexahydro-1,3,4,6,7,11b 2H-pyrazino (2,1-a) isoquinoléinone-4; F = 153-156° C; rendement: 55%;

la (cyclopentylcarbonyl)-2 hexahydro-1,3,4,6,7,11b 2H-pyrazino (2,1-a) isoquinoléinone-4; F = 127-128°C; rendement: 60%;

la (cycloheptylcarbonyl)-2 hexahydro-1,3,4,6,7,11b 2H-pyrazino (2,1-a) isoquinoléinone-4; F = 88-91°C; rendement: 57%;

et respectivement,

la (cyclooctylcarbonyl)-2 hexahydro-1-3,4,6,7,11b 2H-pyrazino (2,1-a) isoquinoléinone-4; F = 107-108°C; rendement: 55%.

*Exemple 3:*

On dissout 7,8 g (0,050 mol) d'acétyl-4 dioxo-2,6 pipérazine dans 200 ml de diméthoxyéthane. On ajoute 3,2 g (0,060 mol) de méthylate de sodium et on abandonne pendant 1 h à température ambiante. Au milieu réactionnel on ajoute, goutte à goutte, sous atmosphère inerte, une solution de 13 g (0,070 mol) de (bromo-2-éthyl) benzène dans 50 ml de diméthoxyéthane et on porte à 85°C pendant 5 h. On évapore à sec, sous vide, et on reprend le résidu par un mélange d'eau et de dichlorométhane. La phase organique décantée est séchée sur sulfate de sodium sec. Le résidu huileux obtenu est purifié par chromatographie sur colonne de silice (éluant: toluène/acétate d'éthyle 7/3). On obtient ainsi l'acétyl-4 dioxo-2,6 phénéthyl-1 pipérazine qui, après recristallisation dans un mélange acétate d'éthyle/isopropanol, fond à 130°C.

On dissout le produit ainsi obtenu dans 500 ml d'éthanol. On ajoute à 0°C, sous atmosphère inerte, 7,15 g (0,055 mol) de $CoCl_2$ et on abandonne pendant 1 h à cette température. On ajoute par portions au milieu réactionnel, à 0°C, 9,5 g (0,25 mol) de borohydrure de sodium et on abandonne pendant 45 min à 0°C. On détruit par l'acétone l'excès de réducteur, on filtre les sels insolubles et on évapore le filtrat à sec. Le résidu obtenu est traité par un mélange d'eau et de dichlorométhane. La phase organique est décantée, puis séchée sur sulfate de sodium sec et évaporée à sec. On purifie le résidu obtenu par filtration sur lit de silice (éluant: acétate d'éthyle), on ajoute par portions le produit ainsi obtenu à 150 ml d'acide chlorhydrique 12N préalablement refroidi à 0°C et on abandonne une nuit à température ambiante. On verse le milieu réactionnel dans l'eau glacée et on extrait avec du dichlorométhane. La phase organique est séchée sur du sulfate de sodium sec, et évaporée à sec. On obtient ainsi l'acétyl-2 hexahydro-1,3,4,6,7,11b 2H-pyrazino (2,1-a) isoquinoléinone-4, qui, après recristallisation dans un mélange d'éther de pétrole et d'acétone, fond à 140-141°C. Rendement global: 60%.

*Exemple 4:*

On dissout 12 g (0,055 mol) de benzoyl-4 dioxo-2,6 pipérazine dans 200 ml de diméthylformamide. On ajoute 3,5 g (0,064 mol) de méthylate de sodium et on abandonne pendant 1 h à température ambiante. Au milieu réactionnel on ajoute, goutte à goutte, sous atmosphère inerte, une solution de 16 g (0,080 mol) de (méthanesulfonyl-2 éthyl) benzène dans 50 ml de diméthylformamide et on porte à 70°C pendant 5 h. On évapore à sec, sous vide, et on reprend le résidu par un mélange d'eau et de dichlorométhane. La phase organique décantée est séchée sur sulfate de sodium sec. L'évaporation du solvant laisse un résidu huileux que l'on purifie par chromatographie sur colonne de silice (éluant: toluène/acétate d'éthyle 7/3). On obtient ainsi la benzoyl-4 dioxo-2,6 phénéthyl-1 pipérazine qui, après recristallisation dans un mélange cyclohexane/acétate d'éthyle, fond à 102°C.

On dissout 16,1 g (0,050 mol) du produit ainsi obtenu dans 600 ml d'éthanol. Sous atmosphère inerte, à 0°C, on ajoute 8,94 g (0,055 mol) de chlorure ferrique et on abandonne pendant 1 h à 0°C. On ajoute par petites quantités, au milieu réactionnel maintenu à 0°C, 9,5 g (0,25 mol) de borohydrure de sodium et on abandonne pendant 1 h à 0°C. L'excès de réducteur est détruit par addition d'acétone. On filtre les sels insolubles et on évapore à sec le filtrat. Le résidu obtenu est traité par un mélange d'eau et de dichlorométhane. La phase organique est décantée, séchée sur sulfate de sodium sec, et on évapore à sec. Le résidu est purifié par filtration sur un lit de silice (éluant: acétate d'éthyle). On ajoute ensuite par petites quantités 10,78 g (0,035 mol) du produit ainsi obtenu à 150 ml d'acide chlorhydrique 12N préalablement refroidi à 0°C et on abandonne une nuit à température ambiante. On verse le milieu réactionnel dans l'eau glacée et on extrait avec du dichlorométhane. La phase organique est séchée sur du sulfate de sodium sec, évaporée à sec. On obtient ainsi la benzoyl-2 hexahydro-1,3,4,6,7,11b 2H-pyrazino (2,1-a) isoquinoléinone-4, F = 160-162°C; rendement global: 58%.

*Exemple 5:*

On dissout sous atmosphère inerte 15 g (0,050 mol) de parabromobenzoyl-4 dioxo-2,6 pipérazine dans 300 ml de diméthylformamide. On ajoute 3,25 g (0,060 mol) de méthylate de sodium et abandonne 1 h 30 à température ambiante. Au milieu réactionnel, on ajoute, goutte à goutte, une solution de 21 g (0,090 mol) d'(iodo-2 éthyl) benzène dans 50 ml de diméthylformamide et porte à 120°C pendant 8 h.

On refroidit puis on verse le milieu réactionnel sur 3000 ml d'eau glacée, extrait 3 fois à l'acétate d'éthyle. Les phases organiques réunies sont lavées à l'eau pour éliminer le diméthylformamide résiduel, séchées sur sulfate de sodium et évaporées à sec. Le résidu huileux obtenu est cristallisé dans l'éther isopropylique, filtré et séché sous vide pour donner la parabromobenzoyl-4 dioxo-2,6 phénéthyl-1 pipérazine qui se présente sous forme de cristaux gris. F = 110-120°C.

On dissout 14 g (0,035 mol) sous atmosphère inerte du produit ainsi obtenu dans 500 ml d'éthanol et on refroidit l'ensemble à 0°C. On ajoute alors 6,5 g (0,038 mol) de chlorure cuivrique dihydraté et on maintient à 0°C pendant 1 h. On ajoute par petites portions 6,6 g (0,175 mol) de $NaBH_4$ en maintenant une température inférieure à 5°C et on abandonne le milieu pendant 45 min à 0°C. On détruit ensuite l'excès de réducteur par l'acétone,

on filtre les sels insolubles puis on évapore à sec le filtrat.

Le résidu huileux obtenu est traité par un mélange de dichlorométhane et d'eau. La phase organique est décantée, séchée sur sulfate de sodium et évaporée à sec.

On purifie le résidu par filtration sur un lit de silice (éluant: acétate d'éthyle) et on évapore à sec. La parabromobenzoyl-4 hydroxy-2 oxo-6 phénéthyl-1 pipérazine cristallise dans l'éther isopropylique sous forme de cristaux blancs. F = 136-138°C.

On ajoute par petites quantités 9,9 g (0,024 mol) du produit obtenu ci-dessus à 150 ml d'acide chlorhydrique 12N refroidi à 0°C et on abandonne une nuit à température ambiante.

On verse le milieu dans l'eau glacée et on extrait au chlorure de méthylène. La phase organique est séchée sur sulfate de sodium et évaporée à sec. Le résidu huileux qui cristallise lentement est recristallisé dans un mélange hexane-acétone pour donner 6,6 g de parabromobenzoyl-2 hexahydro-1,3,4,6,7,11b 2H-pyrazino (2,1-a) isoquinoléinone-4. F = 204-206°C (Köfler); rendement à partir de la parabromobenzoyl-4 dioxo-2,6 pipérazine: 35%.

**Revendications** pour les Etats contractants BE, CH, DE, FR, GB, IT, LI, LU, NL, SE

1. Procédé de préparation d'acyl-2 hexahydro-1,3,4,6,7,11b 2H-pyrazino (2,1-a) isoquinoléinones-4 de formule:

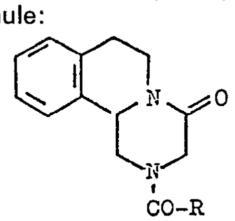

dans laquelle R représente un groupe alkyle contenant jusqu'à 6 atomes de carbone, cycloalkyle ayant de 3 à 8 atomes de carbone, un groupe phényle ou phényle substitué par un atome d'halogène, un groupe hydroxy, alcoxy contenant jusqu'à 6 atomes de carbone, amino, alkylamino contenant jusqu'à 6 atomes de carbone, mercapto, alkylthio contenant jusqu'à 6 atomes de carbone, cyano, trifluorométhoxy ou trifluorométhylthio, caractérisé en ce que:

a) on traite une acyl-4 dioxo-2,6 pipérazine de formule:

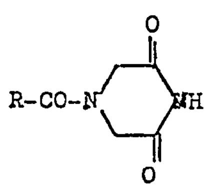

dans laquelle R est tel que défini ci-dessus, par un agent alkylant de formule:

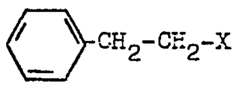

dans laquelle X représente le chlore, le brome, l'iode ou un groupe nucléofuge, en présence d'un agent de condensation alcalin dans un solvant organique inerte,

b) on réduit sélectivement un des groupes carbonyles imidiques du composé ainsi obtenu de formule:

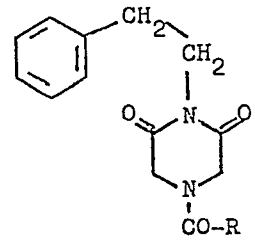

par un hydrure métallique complexe en présence d'un chlorure métallique catalyseur choisi parmi $CuCl_2$, $CoCl_2$, $NiCl_2$, $CrCl_3$, $FeCl_3$, $SnCl_2$, $SrCl_2$, $MnCl_2$, $CeCl_2$, $HgCl_2$, et

c) on cyclise le composé ainsi obtenu de formule:

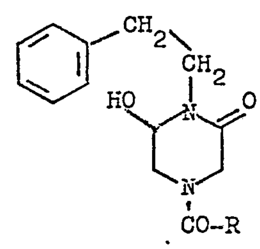

par action d'un acide minéral ou organique fort à une température comprise entre 0 et 25°C.

2. Procédé selon la revendication 1, caractérisé en ce que comme agent alkylant on utilise le chlorure, le bromure ou l'iodure de phénéthyle ou le mésylate ou le tosylate de phénéthyle.

3. Procédé selon la revendication 1, caractérisé en ce que l'agent de condensation alcalin mis en œuvre dans la réaction de condensation de l'étape a est un hydrure de métal alcalin ou un alcoolate de métal alcalin.

4. Procédé selon l'une quelconque des revendications 1 à 3, caractérisé en ce que le solvant de l'étape a est le diméthylformamide, le diméthylacétamide, le dioxanne ou le diméthoxyéthane et la réaction est réalisée sous chauffage à 50-100°C.

5. Procédé selon la revendication 1, caractérisé en ce que l'hydrure métallique complexe utilisé comme réducteur dans l'étape b est un hydrure double d'un métal alcalin et d'un métal alcalino-terreux contenant éventuellement une fraction alkylique.

6. Procédé selon la revendication 5, caractérisé en ce que comme hydrure métallique complexe on utilise le borohydrure de sodium.

7. Procédé selon la revendication 1, caractérisé en ce que comme chlorure métallique catalyseur on utilise le chlorure cuivrique.

8. Procédé selon la revendication 1, caractérisé en ce que l'acide de cyclisation utilisé dans l'étape c est choisi parmi l'acide chlorhydrique, l'acide sulfurique, l'acide phosphorique et l'acide trifluoroacétique.

9. Un composé de formule:

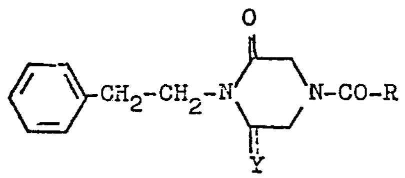

dans laquelle Y représente O ou H,OH et R représente un groupe alkyle contenant jusqu'à 6 atomes de carbone, cycloalkyle ayant 3 à 8 atomes de carbone, phényle ou phényle substitué par un atome d'halogène, un groupe hydroxy, alcoxy contenant jusqu'à 6 atomes de carbone, amino, alkylamino contenant jusqu'à 6 atomes de carbone, mercapto, alkylthio contenant jusqu'à 6 atomes de carbone, cyano, trifluorométhoxy ou trifluorométhylthio.

10. Composé selon la revendication 9, choisi parmi l'acétyl-4 dioxo-2,6 phénéthyl-1 pipérazine, la benzoyl-4 dioxo-2,6 phénéthyl-1 pipérazine, la (cyclohexylcarbonyl)-4 dioxo-2,6 phénéthyl-1 pipérazine et la (bromo-4 benzoyl)-4 dioxo-2,6 phénéthyl-1 pipérazine.

**Revendications** pour l'Etat contractant: AT

1. Procédé de préparation d'acyl-2 hexahydro-1,3,4,6,7,11b 2H-pyrazino (2,1-a) isoquinoléinones-4 de formule:

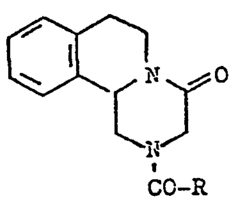

dans laquelle R représente un groupe alkyle contenant jusqu'à 6 atomes de carbone, cycloalkyle ayant de 3 à 8 atomes de carbone, un groupe phényle ou phényle substitué par un atome d'halogène, un groupe hydroxy, alcoxy contenant jusqu'à 6 atomes de carbone, amino, alkylamino contenant jusqu'à 6 atomes de carbone, mercapto, alkylthio contenant jusqu'à 6 atomes de carbone, cyano, trifluorométhoxy ou trifluorométhylthio, caractérisé en ce que:

a) on traite une acyl-4 dioxo-2,6 pipérazine de formule:

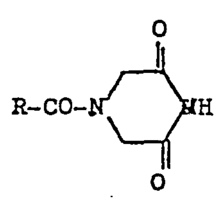

dans laquelle R est tel que défini ci-dessus, par un agent alkylant de formule:

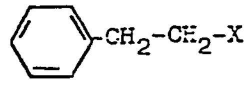

dans laquelle X représente le chlore, le brome, l'iode ou un groupe nucléofuge, en présence d'un agent de condensation alcalin dans un solvant organique inerte;

b) on réduit sélectivement un des groupes carbonyles imidiques du composé ainsi obtenu de formule:

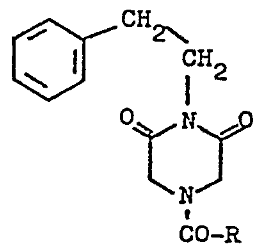

par un hydrure métallique complexe en présence d'un chlorure métallique catalyseur choisi parmi $CuCl_2$, $CoCl_2$, $NiCl_2$, $CrCl_3$, $FeCl_3$, $SnCl_2$, $SrCl_2$, $MnCl_2$, $CeCl_2$, $HgCl_2$, et

c) on cyclise le composé ainsi obtenu de formule:

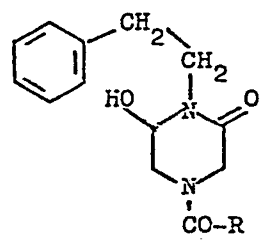

par action d'un acide minéral ou organique fort à une température comprise entre 0 et 25° C.

2. Procédé selon la revendication 1, caractérisé en ce que comme agent alkylant on utilise le chlorure, le bromure ou l'iodure de phénéthyle ou le mésylate ou le tosylate de phénéthyle.

3. Procédé selon la revendication 1, caractérisé en ce que l'agent de condensation alcalin mis en œuvre dans la réaction de condensation de l'étape a est un hydrure de métal alcalin ou un alcoolate de métal alcalin.

4. Procédé selon l'une quelconque des revendications 1 à 3, caractérisé en ce que le solvant de l'étape a est le diméthylformamide, le diméthylacétamide, le dioxanne ou le diméthoxyéthane et la réaction est réalisée sous chauffage à 50-100° C.

5. Procédé selon la revendication 1, caractérisé en ce que l'hydrure métallique complexe utilisé comme réducteur dans l'étape b est un hydrure double d'un métal alcalin et d'un métal alcalino-terreux contenant éventuellement une fraction alkylique.

6. Procédé selon la revedication 5, caractérisé en ce que comme hydrure métallique complexe on utilise le borohydrure de sodium.

7. Procédé selon la revendication 1, caractérisé en ce que comme chlorure métallique catalyseur on utilise le chlorure cuivrique.

8. Procédé selon la revendication 1, caractérisé en ce que l'acide de cyclisation utilisé dans l'étape c est choisi parmi l'acide chlorhydrique, l'acide sulfurique, l'acide phosphorique et l'acide trifluoroacétique.

**Patentansprüche** für die Vertragsstaaten BE, CH, DE, FR, GB, IT, LI, LU, NL, SE

1. Verfahren zur Herstellung von 2-Acyl-1,3,4,6,7,11b-2H-hexahydropyrazino-(2,1-a)-isochinolin-4-onen der Formel:

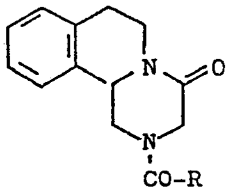

in der R ein bis zu 6 C-Atome enthaltender Alkylrest, Cycloalkyl mit 3 bis 8 C-Atomen, ein Phenylrest oder ein durch ein Halogenatom, eine Hydroxylgruppe, einen Alkoxyrest mit bis zu 6 C-Atomen, eine Aminogruppe, eine Alkylaminogruppe, die bis zu 6 C-Atome enthält, eine Mercaptogruppe, eine bis zu 6 C-Atome enthaltende Alkylthiogruppe, eine Cyanogruppe, eine Trifluoromethoxy- oder Trifluoromethylthiogruppe substituierter Phenylrest ist, dadurch gekennzeichnet, dass man:

a) ein 4-Acyl-2,6-dioxopiperazin der Formel:

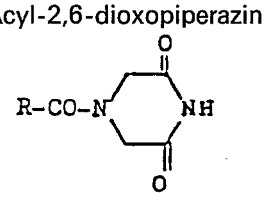

in der R die vorstehend genannten Bedeutungen hat, mit einem Alkylierungsmittel der Formel:

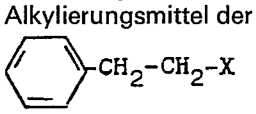

in der X Chlor, Brom, Iod oder eine Nucleofugegruppe (Abgangsgruppe) ist, in Gegenwart eines alkalischen Kondensationsmittels in einem inerten organischen Lösungsmittel behandelt,

b) eine der Imidcarbonylgruppen der so erhaltenen Verbindung der Formel:

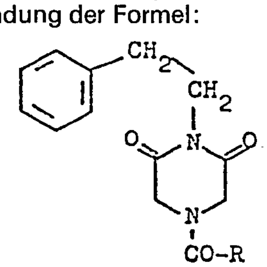

mit einem komplexen Metallhydrid in Gegenwart eines Metallchloridkatalysators, der aus $CuCl_2$, $CoCl_2$, $NiCl_2$, $CrCl_3$, $FeCl_3$, $SnCl_2$, $SrCl_2$, $MnCl_2$, $CeCl_2$, $HgCl_2$ ausgewählt ist, selektiv reduziert und

c) die so erhaltene Verbindung der Formel:

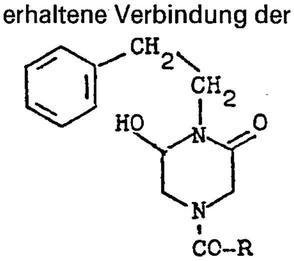

durch Einwirkung einer starken anorganischen oder organischen Säure bei einer zwischen 0 und 25°C liegenden Temperatur cyclisiert.

2. Verfahren nach Anspruch 1, dadurch gekennzeichnet, dass man als Alkylierungsmittel das Chlorid, das Bromid oder das Iodid von Phenethyl oder das Mesylat oder das Tosylat von Phenethyl verwendet.

3. Verfahren nach Anspruch 1, dadurch gekennzeichnet, dass das bei der Kondensationsreaktion der Stufe (a) eingesetzte alkalische Kondensationsmittel ein Alkalimetallhydrid oder ein Alkalimetallalkoholat ist.

4. Verfahren nach irgendeinem der Ansprüche 1 bis 3, dadurch gekennzeichnet, dass das Lösungsmittel der Stufe (a) Dimethylformamid, Dimethylacetamid, Dioxan oder Dimethoxyethan ist und die Reaktion unter Erhitzen auf 50-100°C durchgeführt wird.

5. Verfahren nach Anspruch 1, dadurch gekennzeichnet, dass das in der Stufe (b) als Reduktionsmittel verwendete komplexe Metallhydrid ein doppeltes Hydrid eines Alkalimetalls und eines Erdalkalimetalls, das gegebenenfalls eine Alkylfraktion enthält, ist.

6. Verfahren nach Anspruch 5, dadurch gekennzeichnet, dass man als komplexes Metallhydrid das Natriumborhydrid verwendet.

7. Verfahren nach Anspruch 1, dadurch gekennzeichnet, dass man als Metallchloridkatalysator das Kupfer(II)-chlorid verwendet.

8. Verfahren nach Anspruch 1, dadurch gekennzeichnet, dass die in der Stufe (c) verwendete Cyclisierungssäure aus Salzsäure, Schwefelsäure, Phosphorsäure und Trifluoressigsäure ausgewählt wird.

9. Eine Verbindung der Formel:

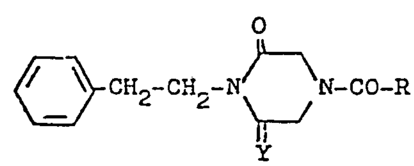

in der Y für O oder H,OH steht und R ein Alkylrest, der bis zu 6 C-Atome enthält, Cycloalkyl mit 3 bis 8 C-Atomen, Phenyl oder ein durch ein Halogenatom, eine Hydroxylgruppe, einen Alkoxyrest mit bis zu 6 C-Atomen, eine Aminogruppe, eine Alkylaminogruppe, die bis zu 6 C-Atome enthält, eine Mercaptogruppe, eine bis zu 6 C-Atome enthaltende Alkylthiogruppe, eine Cyanogruppe, eine Trifluoromethoxy- oder Trifluoromethylthiogruppe substituierter Phenylrest ist.

10. Verbindung nach Anspruch 9, ausgewählt aus dem 4-Acetyl-2,6-dioxo-1-phenethylpiperazin, dem 4-Benzoyl-2,6-dioxo-1-phenethylpiperazin, dem 4-(Cyclohexylcarbonyl)-2,6-dioxo-1-phenethylpiperazin und dem 4-(4-Bromobenzoyl)-2,6-dioxo-1-phenethylpiperazin.

**Patentansprüche** für den Vertragsstaat: AT

1. Verfahren zur Herstellung von 2-Acyl-1,3,4,6,7,11b-2H-hexahydropyrazino-(2,1-a)-isochinolin-4-onen der Formel:

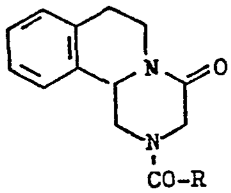

in der R ein bis zu 6 C-Atomen enthaltender Alkylrest, Cycloalkyl mit 3 bis 8 C-Atomen, ein Phenylrest oder ein durch ein Halogenatom, eine Hydroxylgruppe, einen Alkoxyrest mit bis zu 6 C-Atomen, eine Aminogruppe, eine Alkylaminogruppe, die bis zu 6 C-Atome enthält, eine Mercaptogruppe, eine bis zu 6 C-Atome enthaltende Alkylthiogruppe, eine Cyanogruppe, eine Trifluoromethoxy- oder Trifluoromethylthiogruppe substituierter Phenylrest ist, dadurch gekennzeichnet, dass man:

a) ein 4-Acyl-2,6-dioxopiperazin der Formel:

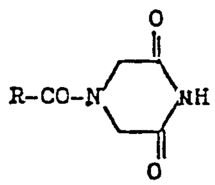

in der R die vorstehend genannten Bedeutungen hat, mit einem Alkylierungsmittel der Formel:

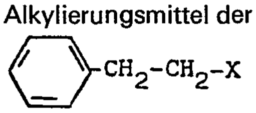

in der X Chlor, Brom, Iod oder eine Nucleofugegruppe (Abgangsgruppe) ist, in Gegenwart eines alkalischen Kondensationsmittels in einem inerten organischen Lösungsmittel behandelt,

b) eine der Imidcarbonylgruppen der so erhaltenen Verbindung der Formel:

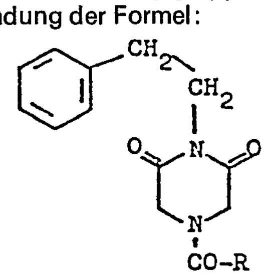

mit einem komplexen Metallhydrid in Gegenwart eines Metallchloridkatalysators, der aus $CuCl_2$, $CoCl_2$, $NiCl_2$, $CrCl_3$, $FeCl_3$, $SnCl_2$, $SrCl_2$, $MnCl_2$, $CeCl_2$, $HgCl_2$ ausgewählt ist, selektiv reduziert und

c) die so erhaltene Verbindung der Formel:

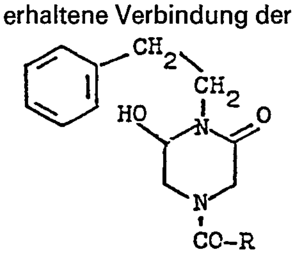

durch Einwirkung einer starken anorganischen oder organischen Säure bei einer zwischen 0 und 25°C liegenden Temperatur cyclisiert.

2. Verfahren nach Anspruch 1, dadurch gekennzeichnet, dass man als Alkylierungsmittel das Chlorid, das Bromid oder das Iodid von Phenethyl oder das Mesylat oder das Tosylat von Phenethyl verwendet.

3. Verfahren nach Anspruch 1, dadurch gekennzeichnet, dass das bei der Kondensationsreaktion der Stufe (a) eingesetzte alkalische Kondensationsmittel ein Alkalimetallhydrid oder ein Alkalimetallalkoholat ist.

4. Verfahren nach irgendeinem der Ansprüche 1 bis 3, dadurch gekennzeichnet, dass das Lösungsmittel der Stufe (a) Dimethylformamid, Dimethylacetamid, Dioxan oder Dimethoxyethan ist und die Reaktion unter Erhitzen auf 50-100°C durchgeführt wird.

5. Verfahren nach Anspruch 1, dadurch gekennzeichnet, dass das in der Stufe (b) als Reduktionsmittel verwendete komplexe Metallhydrid ein doppeltes Hydrid eines Alkalimetalls und eines Erdalkalimetalls, das gegebenenfalls eine Alkylfraktion enthält, ist.

6. Verfahren nach Anspruch 5, dadurch gekennzeichnet, dass man als komplexes Metallhydrid das Natriumborhydrid verwendet.

7. Verfahren nach Anspruch 1, dadurch gekennzeichnet, dass man als Metallchloridkatalysator das Kupfer(II)-chlorid verwendet.

8. Verfahren nach Anspruch 1, dadurch gekennzeichnet, dass die in der Stufe (c) verwendete Cyclisierungssäure aus Salzsäure, Schwefelsäure, Phosphorsäure und Trifluoressigsäure ausgewählt wird.

**Claims** for the Contracting States BE, CH, DE, FR, GB, IT, LI, LU, NL, SE

1. Process for the preparation of 2-acyl-1,3,4,6,7,11b-hexahydro-2H-pyrazino-[2,1-a]-4-isoquinolinones of formula:

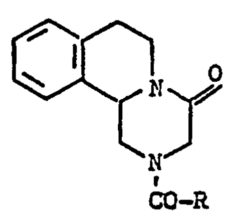

in which R represents an alkyl group containing up to 6 carbon atoms, cycloalkyl having from 3 to 8 carbon atoms or a phenyl or phenyl group substituted by a halogen atom, a hydroxy, an alkoxy containing up to 6 carbon atoms, amino, an alkylamino containing up to 6 carbon atoms, mercapto, alkylthio containing up to 6 carbon atoms, cyano, trifluoromethoxy or trifluoromethylthio group, characterised in that:

(*a*) a 4-acyl-2,6-dioxopiperazine of formula:

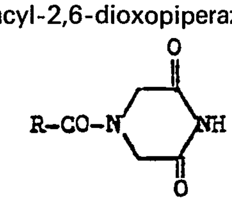

in which R is as defined above, is treated with an alkylating agent of formula:

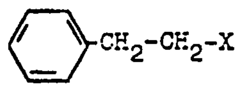

in which X represents chlorine, bromine, iodine or a nucleophobic group, in the presence of an alkali condensation agent in an inert organic solvent,

(*b*) one of the imidic carbonyls of the compound thus obtained of formula:

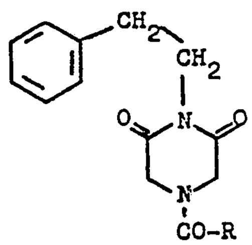

is selectively reduced by a complex metal hydride in the presence of a catalyst metal chloride selected from $CuCl_2$, $CoCl_2$, $NiCl_2$, $CrCl_3$, $FeCl_3$, $SnCl_2$, $SrCl_2$, $MnCl_2$, $CaCl_2$, $HgCl_2$ and

(*c*) the compound thus obtained and of formula :

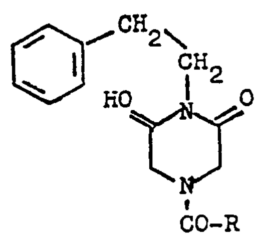

is cyclised by action of a strong organic or mineral acid at a temperature which is between 0 and 25°C.

2. Process according to Claim 1, characterised by the use as alkylating agent of the chloride, bromide or iodide of phenethyl or the mesylate or the tosylate of phenethyl.

3. Process according to Claim 1, wherein the alcali condensation agent used in the condensation reaction of step (*a*) is an alcali metal hydride or alcali metal alcoholate.

4. Process according to any one of Claims 1-3, wherein the solvent of step (*a*) is dimethylformamide, dimethylacetamide, dioxane or dimethoxyethane.

5. Process according to Claim 1, wherein the complex metal hydride used as reducing agent in step (*b*) is a double hydride of an alcali metal or an alcaline-earth metal optionally containing an alkyl moiety.

6. Process according to Claim 5, characterised in that sodium borohydride is used as complex metallic hydride.

7. Process according to Claim 1, characterised in that cupric chloride is used as catalyst metallic chloride.

8. Process according to Claim 1, wherein the acid used in the cyclisation of step (*c*) is selected from hydrochloric acid, sulphuric acid, phosphoric acid or trifluoroacetic acid.

9. A compound of formula:

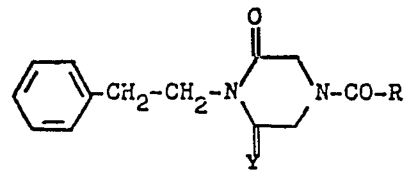

in which Y represents O or H,OH and R represents an alkyl group containing up to 6 carbon atoms, cycloalkyl having from 3 to 8 carbon atoms, phenyl or phenyl group substituted by a halogen atom, a hydroxy, alkoxy containing up to 6 carbon atoms, amino, alkylamino containing up to 6 carbon atoms, mercapto, alkylthio containing up to 6 carbon atoms, cyano, trifluoromethoxy or trifluoromethylthio group.

10. A compound according to Claim 9, selected from 4-acetyl-2,6-dioxo-1-phenethyl piperazine, 4-benzoyl-2,6-dioxo-1-phenethyl piperazine, 4-(cyclohexylcarbonyl)-2,6-dioxo-1-phenethyl piperazine, 4-(cyclohexylcarbonyl)-2-hydroxy-6-oxo-1-phenethyl piperazine and 4-(4-bromobenzoyl)-2,6-dioxo-1-phenethyl piperazine.

**Claims** for the Contracting State: AT

1. Process for the preparation of 2-acyl-1,3,4,6,7,11b-hexahydro-2H-pyrazino-[2,1-a]-4-isoquinolinones of formula:

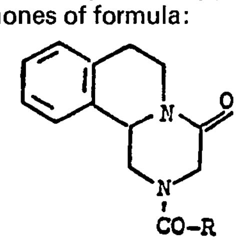

in which R represents an alkyl group containing up to 6 carbon atoms, cycloalkyl having from 3 to 8 carbon atoms or a phenyl or phenyl group substituted by a halogen atom, a hydroxy, an alkoxy containing up to 6 carbon atoms, amino, an alkylamino containing up to 6 carbon atoms, mercapto, alkylthio containing up to 6 carbon atoms, cyano, trifluoromethoxy or trifluoromethylthio group, characterised in that:

(*a*) a 4-acyl-2,6-dioxopiperazine of formula:

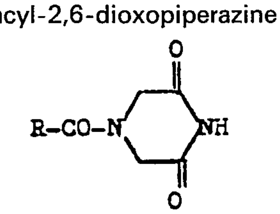

in which R is as defined above, is treated with an alkylating agent of formula:

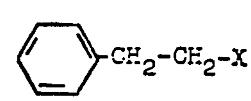

in which X represents chlorine, bromine, iodine or a nucleophobic group, in the presence of an alkali condensation agent in an inert organic solvent,

(*b*) one of the imidic carbonyls of the compound thus obtained of formula:

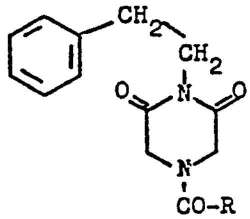

is selectively reduced by a complex metal hydride in the presence of a catalyst metal chloride selected from $CuCl_2$, $CoCl_2$, $NiCl_2$, $CrCl_3$, $FeCl_3$, $SnCl_2$, $SrCl_2$, $MnCl_2$, $CaCl_2$, $HgCl_2$ and

(*c*) the compound thus obtained and of formula:

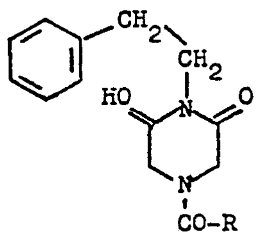

is cyclised by action of a strong organic or mineral acid at a temperature which is between 0 and 25°C.

2. Process according to Claim 1, characterised by the use as alkylating agent of the chloride, bromide or iodide of phenethyl or the mesylate or the tosylate of phenethyl.

3. Process according to Claim 1, wherein the alcali condensation agent used in the condensation reaction of step (*a*) is an alcali metal hydride or alcali metal alcoholate.

4. Process according to any one of Claims 1-3, wherein the solvent of step (*a*) is dimethylformamide, dimethylacetamide, dioxane or dimethoxyethane.

5. Process according to Claim 1, wherein the complex metal hydride used as reducing agent in step (*b*) is a double hydride of an alcali metal or an alcaline-earth metal optionally containing an alkyl moiety.

6. Process according to Claim 5, characterised in that sodium borohydride is used as complex metallic hydride.

7. Process according to Claim 1, characterised in that cupric chloride is used as catalyst metallic chloride.

8. Process according to Claim 1, wherein the acid used in the cyclisation of step (*c*) is selected from hydrochloric acid, sulphuric acid, phosphoric acid or trifluoroacetic acid.